(19) Europäisches Patentamt
European Patent Office
Office européen des brevets

(11) **EP 3 909 969 A1**

(12) **EUROPEAN PATENT APPLICATION**

(43) Date of publication:
**17.11.2021 Bulletin 2021/46**

(51) Int Cl.:
***C07K 7/08*** *(2006.01)*     ***C07K 14/00*** *(2006.01)*

(21) Application number: **20174972.8**

(22) Date of filing: **15.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Freie Universität Berlin**
**14195 Berlin (DE)**

(72) Inventors:
 • **Koksch, Beate**
  **14532 Kleinmachnow (DE)**
 • **Hellmund, Katharina**
  **12305 Berlin (DE)**

(74) Representative: **Maikowski & Ninnemann**
**Patentanwälte Partnerschaft mbB**
**Postfach 15 09 20**
**10671 Berlin (DE)**

(54) **PEPTIDE FOR HYDROGEL FORMATION**

(57) The invention relates to a peptide, comprising n heptad repeat units having a primary structure of LXXLKKE (SEQ ID NO. 1), wherein in one heptad repeat unit one of the X residues is A and the other of the X residues is A, F, Y or W, wherein in n-1 heptad repeat units both X residues are A, wherein n is 2 to 10. This peptide forms a hydrogel that can be used as extracellular matrix for cultivating cells better than currently available products.

FIG 5A

Printed by Jouve, 75001 PARIS (FR)

EP 3 909 969 A1

**Description**

The present invention relates to a peptide according to the preamble of claim 1, to a hydrogel comprising such a peptide according to the preamble of claim 11, to medical uses of such a peptide or such a hydrogel according to the preamble of claim 13, to non-medical uses of such a peptide or such a hydrogel according to the preamble of claim 14, and to a method for manufacturing such a peptide according to the preamble of claim 15.

There is an interest in the development of materials that mimic the biological environment of cells for directing their behavior for medical and clinical applications. Design and development of biomaterials for their application as stem cell culture substrates enabling directing tissue specific differentiations requires a sufficient understanding about cell proliferation and differentiation mechanisms. In this context, numerous different approaches using peptides as cell culture substrates have been successfully investigated.[1] But so far several commonly used substrates have animal-derived ingredients and therefore a high batch-to-batch variability.

Peptide-based biomaterials circumvent such variabilities. US 2011/0189284 A1 relates to a protein fibril comprising a plurality of first peptide monomer units arranged in a first strand and a plurality of second peptide monomer unit arranged in a second strand. Thus, this patent application describes a peptide heterodimer forming a protein fibril.

Peptides and their derivatives can be adjusted with regard to their structural properties by the use of canonical and non-canonical amino acid building blocks in order to achieve complex architectures.[3]

It is an object of the present invention to provide novel compounds being appropriate to be used as biomaterial that overcome the disadvantages of prior art compounds.

This object is achieved with a peptide comprising n heptad repeat units having a primary structure corresponding to the amino acid sequence LXXLKKE (SEQ ID NO. 1). In this context, in one heptad repeat unit one of the X residues is alanine (A) and the other of the X residues is alanine (A), phenylalanine (F), tyrosine (Y) or tryptophan (W). In the remaining n-1 heptad repeat units both X residues are alanine (A). The variable n is an integer between 2 and 10, in particular between 3 and 9, in particular between 4 and 8, in particular between 5 and 7.

Such a peptide can be synthesized with high reproducibility and fine-tuned in order to mimic specific structures. It shows self-assembly properties and can also be denoted as coiled-coil peptide. Coiled coil forming peptides can adopt higher ordered structures that are α-helical in nature. Coiled coil motifs are common among natural proteins,[4] in fact, primary sequence analysis has shown that nearly 2-3 % of all natural proteins contain coiled-coil domains.[4,5] The coiled-coil motif consists of two to seven α-helical strands that form a left-handed superhelical twist.[6,7]

The primary amino acid sequence (primary structure) of the peptide is characterized by a seven residue periodicity, called a heptad repeat.[6] The heptad repeat is denoted by *a-b-c-d-e-f-g.* In coiled coil peptides the positions *a* and *d* are commonly occupied by nonpolar amino acid residues such as leucine, isoleucine, and valine, which form the hydrophobic core[8,9] that directs the folding and packing of the amphipathic structure.[6]

Positions *e* and *g* are occupied mostly by charged amino acids like arginine, glutamic acid and lysine that provide intermolecular ionic interactions within the bundle.[7,10]

The remaining solvent exposed positions *b, c* and *f* are directed toward the outside of the bundle and are appropriate for modifications, e. g., by carbohydrate moieties, peptide ligands, small organic drug molecules or dyes that might be used as diagnostic tools.[7,11,12]

The peptides according to the present invention also exhibit these general structural motives. However, they surprisingly show unexpected superior properties with respect to hydrogel formation so that the peptides according to the present invention are particularly well appropriate to be used as biomaterial such as basic material for a synthetic extracellular matrix.

In an embodiment, the heptad repeat units have a primary structure corresponding to the amino acid sequence LAXLKKE (SEQ ID NO. 2). In this context, in one heptad repeat unit the X residue is alanine (A), phenylalanine (F), tyrosine (Y) or tryptophan (W), wherein in the remaining n-1 heptad repeat units the X residue is alanine (A).

In an embodiment, the heptad repeat units have a primary structure corresponding to the amino acid sequence LXALKKE (SEQ ID NO. 3). In this context, in one heptad repeat unit the X residue is alanine (A), phenylalanine (F), tyrosine (Y) or tryptophan (W), wherein in the remaining n-1 heptad repeat units the X residue is alanine (A).

In an embodiment, the heptad repeat units have a primary structure corresponding to the amino acid sequence LAALKKE (SEQ ID NO. 4).

In an embodiment, the peptide comprises 1 to 10, in particular 2 to 9, in particular 3 to 8, in particular 4 to 7, in particular 5 to 6 additional amino acids. These additional amino acids can be present between individual heptad repeat units and/or at the N-terminus and/or at the C-terminus of the peptide.

In an embodiment, the additional amino acids comprise additional N-terminal amino acids and additional C-terminal amino acids. In this embodiment, a number of the additional N-terminal amino acids is greater than a number of the additional C-terminal amino acids. Expressed in other words, there are more additional N-terminal amino acids than additional C-terminal amino acids. To give an example, the number of additional N-terminal amino acids can be 3 to 8, in particular 4 to 7, in particular 5 to 6, wherein the number of additional C-terminal amino acids is 1 to 4, in particular 2 to 3, provided that the total number of additional amino acids is not bigger than 1 to 10.

In an embodiment, all heptad repeat units are directly linked to each other. Thus, in this embodiment there are no additional amino acids between the individual heptad repeat units. Rather, if additional amino acids are present in this embodiment, they can only be present at the N-terminus and/or the C-terminus of the peptide.

In an embodiment, the peptide has a primary structure corresponding to the amino acid sequence LKKELXXLKKELXXLKKELXXLKKEL (SEQ ID NO. 5). In this context, one of the X residues is alanine (A), phenylalanine (F), tyrosine (Y) or tryptophan (W), wherein all other of the X residues are alanine (A). Here, the amino acids LKKE represent additional N-terminal amino acids, and the amino acid L represents an additional C-terminal amino acids, wherein the peptide comprises 3 heptad repeat units having a primary structure corresponding to the amino acid sequence LXXLKKE.

In an embodiment, the peptide has a primary structure corresponding to the amino acid sequence LKKELAALKKELAALKKELAALKKEL (SEQ ID NO. 6).

In an embodiment, individual amino acids of the precedingly mentioned amino acid sequences can be functionalized by a specific ligand. In another embodiment, no such functionalization is present so that the amino acid sequences can be considered as bare amino acid sequences.

In an embodiment, the peptide comprises at least one ligand molecule covalently bound to any of the amino acids of the peptide. The ligand molecule is chosen from the group consisting of peptides, molecules comprising an epitope, hydrocarbons, carbohydrates, and pharmaceutically active compounds. An RGD peptide (comprising or consisting of the amino acid sequence arginine, glycine and aspartic acid) is a particular appropriate peptide ligand to be conjugated to the peptide. Mannose, galactose, and sialic acids are particularly appropriate carbohydrates. Small organic molecules are particularly appropriate pharmaceutically active compounds. Particularly appropriate small molecules are chemically modified modulators which were previously reported to be involved in the development of stem cells, e.g. serotonin for enhancing cell expansion and vitamin D3 for osteogenic differentiation of mesenchymal stem cells. [45] The epitope can also be denoted as recognition motif of an antigen. It is a motif recognized by an antibody.

If the peptide is synthesized by solid phase peptide synthesis, it is possible to directly synthesize this ligand together with the peptide on the solid phase. This can be done by using modified amino acids for solid phase peptide synthesis. This approach significantly facilitates the manufacturing process of the peptide.

In an embodiment, the peptide comprises exactly one ligand per peptide molecule. In another embodiment, the peptide comprises one ligand per heptad repeat unit.

In an embodiment, the peptide comprises 2, 3, 4, 5, 6, 7, or 8 ligands per peptide molecule.

In an embodiment, the ligand is covalently bound only to the lysine side chain in f position (i.e., in the sixth position) of the heptad repeat unit of the peptide.

In an aspect, the present invention relates to a hydrogel comprising a plurality of molecules of a peptide according to the preceding explanations. The plurality of peptide molecules is present in a coiled coil arrangement or coiled coil superstructure. Such a coiled coil can comprise up to seven single strands, wherein at least 2 individual strands forming a homodimer are necessary for such a coiled coil configuration. Each individual strand shows a helical secondary structure. In an embodiment, the coiled coil comprises 2 to 7, in particular 3 to 6, in particular 4 to 5 individual strands of the peptide.

In an embodiment, all peptide molecules in the hydrogel have the same primary structure. In an embodiment, some peptide molecules in the hydrogel comprise a bare amino acid primary sequence (i.e., amino acids without any functionalization), whereas other peptide molecules in the hydrogel comprise a primary sequence comprising at least one functionalized amino acid per peptide molecule. By mixing peptide molecules having a bare amino acid primary sequence and peptide molecules comprising functionalized amino acids, it is possible to individually adjust the type and/or degree of functionalization in the hydrogel. Thus, it is possible to design a chemically defined network with adjustable peptide concentration, adjustable ligand density and/or adjustable ligand types.

Expressed in other words, the composition of the hydrogel can be adapted to the desired application. E.g., the water content, the degree of functionalization, the type of functionalization, the stiffness, the flexibility, and other mechanic properties of the hydrogel can be adjusted over a wide range.

In an aspect, the present invention relates to the medical use of the peptide according to the preceding explanations or of the hydrogel according to the preceding explanations. This medical use particularly relates to use in therapy or surgery. To give an example, the peptide of the hydrogel can be used as implant, for enhancing wound healing, as replacement for cartilage or more generally as injectable hydrogel for curative purposes. In an embodiment, the peptide or hydrogel is used for inhibiting viruses such as an influenza virus or a corona virus by interacting with the virus over a ligand (in particular a carbohydrate) covalently bound to the peptide and interacting with a recognition site on the surface of the virus.

In an aspect, the present invention relates to the use of the peptide according to the preceding explanations or of the hydrogel according to the preceding explanations for manufacturing cosmetics and pharmaceuticals such as ointments or creams for cosmetic purposes or for pharmaceutical purposes.

In an aspect, the present invention relates to the use of the peptide according to the preceding explanations or of the hydrogel according to the preceding explanations as extracellular matrix for in vitro applications. In particular, if the peptide carries at least one functionalization, it can be well used for mimicking the extracellular matrix and can thus well be used for cultivating cells such as stem cells in vitro. The functionalization can comprise a recognition sequence for cells, e.g., to direct stem cell proliferation and differentiation. Thus, the peptide or hydrogel can well be used for tissue engineering applications or for applications of regenerative medicine. In an aspect, the present invention relates to the use of the hydrogel according to the preceding explanations as a screening platform for an examination of the kind and the density of ligands presented within the three-dimensional structure of the hydrogel.

In an aspect, the present invention relates to a method for manufacturing a peptide according to the preceding explanations. This method comprises the steps explained in the following.

In a first step, a peptide is synthesized by solid phase peptide synthesis on a solid phase peptide synthesis resin. The peptide comprises n heptad repeat units having a primary structure of LXXLKKE (SEQ ID NO. 1). In one heptad repeat unit one of the X residues is alanine (A) and the other of the X residues is alanine (A), phenylalanine (F), tyrosine (Y) or tryptophan (W). In the remaining n-1 heptad repeat units both X residues are alanine (A), wherein n is 2 to 10.

After successful synthesis, the synthesized peptide is cleaved from the solid phase peptide synthesis resin.

Finally, the synthesized and cleaved peptide can be optionally purified by standard purification techniques.

In an embodiment, at least one ligand-modified amino acid, in particular ligand-modified lysine, is used in addition to non-modified amino acids for solid phase peptide synthesis. In doing so, it is possible to directly introduce ligands into the synthesized peptide without a further functionalization step.

In an embodiment, at least one amino acid modified by a protective group, in particular modified by an orthogonal protective group, in particular a lysine carrying a protective group such as an orthogonal protective group, is used in addition to non-modified amino acids for solid phase peptide synthesis. In doing so, it is possible to separately remove such protective group so that a subsequent functionalization of the amino acid with a ligand is possible. A peptide or a sugar molecule is an appropriate ligand. Even though this procedure requires a separate functionalization step, this might work in some instances (depending on the structure of the ligand) better than a direct functionalization.

All embodiments of the described peptide can be combined in any desired way and can be transferred either individually on any arbitrary combination to the hydrogel, the uses and the manufacturing method. Likewise, all embodiments of the described hydrogel can be combined in any desired way and can be transferred either individually or in any arbitrary combination to the described peptide, the described uses and the described manufacturing method. All embodiments of the described uses can be combined in any desired way and can be transferred either individually or in any arbitrary combination to the respective other uses, to the described peptide, to the described hydrogel and to the described manufacturing method. All embodiments of the manufacturing method can be combined in any desired way and can be transferred either individually or in any arbitrary combination to the described peptide, the described hydrogel and the described uses.

Further details of aspects of the present invention will be described in the following with respect to exemplary embodiments and accompanying Figures. In the Figures:

Figure 1A     shows the CD spectra of 0.5 % by weight (wt%) pure peptide hydrogels directly after sample preparation at 37°C;

Figure 1B     shows the CD spectra of 0.5 wt% peptide hydrogel mixtures directly after sample preparation at 37°C;

Figure 2A     shows the moduli G' and G" of different concentrations of pure peptide at 25 °C;

Figure 2B     shows the moduli G' and G" of different concentrations of a peptide mixture at 25 °C;

Figure 2C     shows the moduli G' and G" of different concentrations of pure peptide at 37 °C;

Figure 2D     shows the moduli G' and G" of different concentrations of a peptide mixture at 37 °C;

Figure 3A     shows the phase angle of pure peptide at different concentrations at 25 °C;

Figure 3B     shows the phase angle of a peptide mixture at different concentrations at 25 °C;

Figure 3C     shows the phase angle of pure peptide at different concentrations at 37 °C;

Figure 3D     shows the phase angle of a peptide mixture at different concentrations at 37 °C;

Figure 4A     shows the scattering patterns of pure peptide at different concentrations;

Figure 4B    shows the scattering patterns of a peptide mixture at different concentrations;

Figure 5A    shows the viability profiles of seeded NIH3T3 cells on peptide hydrogels after 24 h; and

Figure 5B    shows the viability profiles of seeded NIH3T3 cells on peptide hydrogels after 72 h.

The Figures will be explained in the following with respect to exemplary embodiments.

A peptide sequence called hFF03 and corresponding to SEQ ID NO. 6 was designed that self-assembles into stable α-helical fibers and builds up self-supporting hydrogels under physiological conditions. This peptide hFF03 was conjugated with a tripeptide ligand, RGD, as well as with the monosaccharide mannose to result in a library of three peptides (non-modified, RGD-modified and mannose-modified) in order to generate peptide conjugates that resemble an artificial extracellular matrix. Conjugation of these molecules was achieved by using a previously reported all-on-solid-phase (AOSP) approach, where RGD-sequence and mannose were included by standard solid-phase peptide synthesis.[11]

The peptides were combined in a selected ratio and concentration out of stock solutions in hexafluoroisopropanol. After evaporation of the solvent, the peptides were dissolved in buffer solution or cell culture medium. Fiber-formation occurs as a consequence of self-assembly of the peptides leading to hydrogel formation. Hydrogel formation can be tested initially by inversion of the sample vial containing the peptides.[2]

## Materials and Methods

Solid-phase peptide synthesis was performed with resins from Novabiochem. hFF03 and hFF03 variants were synthesized using preloaded Fmoc-Leu-NovaSyn TGA resin (0.2 mmol/g amino acid loading) respectively. Synthesis was performed by standard Fmoc/tBu chemistry on 0.1 mmol scale. Fluorenylmethylcarboxycarbonyl (Fmoc)-protected amino acids were purchased from Orpegen. Fmoc-deprotection was performed with 2 % piperidine and 2 % 1,8-Diazabicyclo[5.4.0]undec-7-en (DBU) in dimethylformamide (DMF) (3x7 min with 5 mL of deprotecting solution) at each step.

Synthesis of hFF03 and hFF03-variants accomplished in two steps: hFF03 was synthesized in 2 h double couplings using an Activo-P11 Automated Peptide Synthesizer with eight-fold excess of amino acid, 2-(1H-7-Azabenzotriazol-1-yl)-1,1,3,3-tetramethyluronoium hexafluorphosphate (HATU) as coupling agent, and sixteen-fold excess of N,N-diisopropylethylamin (DIPEA) relative to resin loading. In case of decorated variants of hFF03 tBu-protected lysine in position 17 was substituted by N-Methyltrityl-protected (Mtt) lysine purchased from Carbolution. This protecting group is removable under mild acidic conditions. After full-length synthesis of the peptides, Boc-aminobenzoic acid (Abz), purchased from Bachem, was coupled to N-terminus of each peptide to block it for further synthesis steps. Further synthesis of hFF03-variants was performed by selective deprotection of lysine side chain in position 17 by treatment with a solution containing 1 % trifluoroacetic acid (TFA) (v/v) and 1 % methanol (MeOH) (v/v) in dichloromethane (DCM) according to literature established protocols. The free amine group of lysine side chain is used as starting point for further coupling of ligands.

In case of the tripeptide RGD, manual amino acids couplings using eight-fold excess of amino acid, HATU and sixteen-fold excess of DIPEA relative to resin loading were performed. Fmoc-deprotection was performed at each step as described above.

In case of mannose-functionalization of $NH_2$ of lysine side-chain in position 17, amino functionality was switched to carboxy-functionality using glutaric anhydride in three-fold excess and catalytic amount of DIPEA for three hours. Afterwards carboxy function was activated using (1-Cyano-2-ethoxy-2-oxoethylidenaminooxy)dimethylamino-morpholino-carbenium hexafluorophosphate (COMU) in three-fold excess and six -old excess of DIPEA. Directly after activation 1-amino-1-deoxy-mannopyranose (Santa Cruz) was coupled to carboxy function.

Full cleavage of all peptides was performed using 10 mL of cleavage cocktail containing 95 % TFA, 3 % $H_2O$ and 2 % triisopropylsilane (TIS) for 3,5 h. Peptides were precipitated using ice cold diethylether. After decantation of ether, peptides were resolved in water and lyophilized. Purification of peptides was achieved using preparative HPLC.

## Exchange of TFA against chloride

TFA was exchanged against chloride according to literature established protocols.[41] Peptides were dissolved in water at a concentration of 0.52 mM. Afterwards 6 M HCl was mixed to peptide solutions to give final concentration of 7.5 mM HCl. The solutions were allowed to stir at room temperature for 1 minute and lyophilized. This procedure was repeated 5 times.

**Dialysis of peptides**

Dialysis of peptides was achieved by Spectra/Por® Float-A-Lyzer® (Carl Roth) with molecular weight cut-off of 100-500 Da and performed according to suppliers instructions. Peptides were dialyzed against water over three days. Water was changed thrice a day. After completion peptide solutions were freeze dried.

**Sample preparation**

Pure peptides were dissolved in 1 mL 1,1,1,3,3,3-Hexafluoroisopropanol (HFIP) and sonicated for 15 min. Peptide concentration was determined by UV-spectroscopy at 320 nm (Abz). An aliquot of the stock solution was briefly evaporated in gentle N2-stream and dissolved in 1 mL of D-PBS containing 6 M of guanidine hydrochloride. Absorbance at 320 nm of this solution was measured using a Varian Cary 50 photometer (Varian Medical Systems, Palo Alto, CA, USA). The concentrations of stock solutions were calculated upon a calibration curve of Abz-Gly-OH. For certain peptide concentration required aliquots were completely evaporated and dissolved in buffer solution. In case of hydrogel-mixtures aliquots of desired peptide stock-solutions were mixed before evaporation. After evaporation peptides were dissolved in D-PBS (Lonza, w/o $Mg^{2+}$, $Ca^{2+}$) or DMEM (Lonza, 4.5 g/L glucose).

**CD spectroscopy**

CD spectra of peptide hydrogels were recorded using a Quartz Suprasil® cuvette with detachable windows and a path length of 0.1 mm (Hellma Analytics, Müllheim, Germany). Measurements were performed at 37°C and are the mean of 3 independent measurements. CD spectra are background-corrected by subtraction of buffer spectra at 37°C.

**Cryo-Transmission Electron Microscopy (cryo-TEM)**

Cryo-TEM were measured using 0.15 wt% peptide hydrogel samples. 5 $\mu$l aliquots of the respective peptide gels were applied to pre-cleaned 200 mesh perforated carbon film-covered microscopic grids (R1/4 batch of Quantifoil, MicroTools GmbH, Jena, Germany). The grids were cleaned with chloroform and hydrophilized by 60 s glow discharging at 8 W in a BALTEC MED 020 device (Leica Microsystems, Wetzlar, Germany). Vitrifying of the samples occurred by automatic blotting and plunge freezing with a FEI Vitrobot Mark IV (Thermo Fisher Scientific Inc., Waltham, Massachusetts, USA) using liquid ethane as cryogen. The vitrified samples were transferred to the autoloader of a FEI TALOS ARCTICA electron microscope (Thermo Fisher Scientific Inc., Waltham, Massachusetts, USA). The microscope is equipped with a high-brightness field-emission gun (XFEG), which operates at an acceleration voltage of 200 kV. Acquisition of the micrographs was carried out on a FEI Falcon 3 direct electron detector (Thermo Fisher Scientific Inc., Waltham, Massachusetts, USA) using a 70 $\mu$m objective aperture at a nominal magnification of 28,000 or 36,000 x, corresponding to a calibrated pixel size of 3.69 or 2.97 Å/pixel, respectively.

**Rheology**

All rheological measurements were performed using a temperature-controlled Bohlin Gemini 200 HR Nano rheometer. The samples were measured at room temperature T1 = 25°C and at the physiological temperature $T_2$ = 37°C. All experiments were conducted using a plate-plate geometry with the upper rotating plate having a diameter of 40 mm. The gap size between the two plates was kept constant at 200 $\mu$m. To avoid evaporation effects the setup of sample and confining geometry was surrounded by a solvent trap. For reasons of reproducibility, all rheological experiments were repeated three times. From these triplicate measurements the error bars were estimated and found to be in the range of 10 to 20 % of the measured values.

**Small angle neutron scattering (SANS)**

The SANS experiments were performed at Helmoltz Zentrum Berlin (HZB, Berlin, Germany) with the spectrometer V4 and at Laboratoire Leon Brillouin (LLB, Saclay, France) with the spectrometer PAXY. For the measurements at V4 spectrometer (HZB) three different sample-detector-distances D1 = 1.35 m, D2 = 6.75 m and $D_3$ = 16.0 were used. The wavelength for $D_1$ and D2 was set at 4.5 Å while for $D_3$ a wavelength of 10.03 Å was used. This enabled covering a q-range of 0.02 - 6.5 $nm^{-1}$, where $q = 4\pi \sin(\theta/2)/\lambda$ is the scattering vector with $\theta$ being the scattering angle. Also for the measurements at PAXY spectrometer (LLB) three sample-detector-distances $D_1$ = 1 m, $D_2$ = 5 m and $D_3$ = 6.7 m were used, whereby for $D_1$ and $D_2$ the wavelength was set to 4 Å and for $D_3$ to 12 Å allowing to cover a q-range of 0.04 - 6.3 $nm^{-1}$similar to the experiments at V4. The have comparable experimental conditions for all measurements 2 mm Hellma QS 110 cuvettes were used throughout. During the experiments all samples are kept at 25°C.To reduce the two-dimensional detector images to one-dimensional datasets the BerSANS software[42] was used for the data collected at V4, while for the data recorded at PAXY the PASiNET software[43] was used. For both reduction methods the one-dimensional data were obtained as differential cross sections by taking into account the samples transmission and comparing the scattering intensities to the one of a $H_2O$ sample with 1 mm thickness.

## Cytotoxicity of different hydrogel compositions

### Cell Culture

NIH/3T3 embryonic mouse fibroblast cells were cultured in DMEM culture medium (Lonza, 4,5 g/L glucose, 10 % FCS, 1 % penicillin/streptomycin) in a humidified incubator (5 % $CO_2$, 37°C). The cells were grown in 175 cm$^2$ cell culture flaks and medium was changed thrice a week. Upon confluency of 70 % - 80 % the culture was subcultured according to the detected cell number.

### Cytotoxicity assay of hydrogel cultured NIH/3T3 cells by use of Cell Counting Kit-8 (CCK-8)

To determine whether different peptide hydrogel compositions would be tolerated by the cells CCK-8 was used to conduct viability of cells during a 3 days cell culture. A suspension of NIH/3T3 cells in DMEM (4.5 g/L, 10 % FCS, 1 % penicillin/streptomycin) was seeded in a transparent 96-well plate with a density of 10.000 cells in an overall volume of 100 $\mu$L per well containing the respecting hydrogels. 10 $\mu$L of 1 % (w/v) SDS was added to the respective wells of positive control and the plate was further incubated for 24 h and 72 h at 37°C and 5 % $CO_2$ respectively. After each time points 10 $\mu$L of CCK-8 solution were added to each well and allowed to incubate for 2 h in a humidified incubator at 37°C. During this time dehydrogenases in viable cells reduces WST-8 tetrazolium to formazan, which will be detected by measuring the absorbance at 450 nm. Absorbance was measured by using a Tecan Infinite 200 Pro microplate reader. Three experiments were performed in triplicates per hydrogel candidate (n=3).

### Results and Discussion

### Extracellular Matrix design

The development of tailorable and biocompatible 3D substrates that make possible to influence cell behavior *in vitro* is of high interest for a variety of cell-based applications in the field of tissue engineering and regenerative medicine. Within the framework of the present disclosure, a tunable peptide-based 3D material was developed to build a highly complex cellular environment. This tunable peptide hydrogel bears different functionalities at one scaffold and enables the imitation of native extracellular environment for different stages of cellular development. The cellular behavior can be influenced by structural and functional features, as for example by the stiffness of the cell culture substrate. These properties play also a fundamental role in the recognition and release of signal cascades.

The described substrate is able to accomplish these features simultaneously. In addition, functional ligands can be presented on the scaffold in a multivalent fashion. In the presented study, the newly hydrogelating peptide hFF03 was chosen as a scaffold that forms stable hydrogels while multivalently presenting a variety of different functional ligands simultaneously. hFF03 variants were preassembly functionalized according to known synthesis protocols.[11] In order to design a variety of different extracellular matrices, the newly designed coiled coil scaffold hFF03 was conjugated with different functional ligands. These conjugates were mixed with the undecorated peptide scaffold (wild type hydrogelator, hFF03) in different ratios. The ligands are then distributed within the three-dimensional structure of the hydrogel. This approach allowed for a fine-tuning of the concentration of the functional ligands and, thus, for a fine-tuning of the properties of the resulting functionalized hydrogels that formed an artificial extracellular matrix (ECM) for cell culture experiments.

The design of the studied peptide scaffold hFF03 follows the rules of coiled-coil formation which are described above. hFF03 consists of three repeating heptads and an additional hydrophobic part at its C-terminus.[11] "Sticky ends" are created by half a heptad at the N-terminus of the peptides to trigger fiber-assembly.[8,11] Leucine residues are building the hydrophobic core of the peptides, whereas the stabilization of the $\alpha$-helical structure occurs by positively and negatively charged amino acids lysine and glutamic acid in positions e and g. Furthermore, *b* and *c* positions are occupied with alanine residues to shield the solvent-exposed domain of the peptide[21] and differs in this regard from the original FF03 sequence.[11]

Solvent-exposed positions *f* of hFF03 are substituted with lysines. Instead of the commonly used boc-protected lysine a methyltrityl-protected (Mtt) lysine in position 17 (Mtt). The Mtt-group can selectively be removed[25] to generate a free amine functionality for the built-up of different functional ligands as for example small peptides by orthogonal solid-phase peptide synthesis. For carbohydrate-conjugation ligand, the amino-group was switched to a carboxy-functionality by means of glutaric anhydride.[11] By activation this free carboxylic acid function on resin the amino-functionalized carbohydrate was coupled. Based on this strategy, a peptide library containing the undecorated peptide scaffold hFF03, a RGD-functionalized hFF03-K17-RGD, and a mannose-functionalized hFF03-K17-Man were synthesized all on solid-phase (see Table 1). Functionalization with the tripeptide RGD was chosen, because it is a well-known recognition motif to promote adhesion of cells and is presented by fibronectin and collagen in native ECMs.[26,27] To proof whether sugar-coupling interferes with the structure and hydrogel formation of the peptide, mannose was chosen. The purity of the peptides was verified by HPLC, UHPLC and MS.

Table 1. Synthesized peptide variants. Bold K represents ligand bearing position.

| Name | Sequence/Ligand |
| --- | --- |
| hFF03 | LKKELAALKKELAALK**K**ELAALKKEL |
| hFF03-K17-Man | Mannose-moiety presented by lysine 17 of hFF03-scaffold |
| hFF03-K17-RGD | RGD-sequence presented by lysine 17 of hFF03-scaffold |

This rational design enables the combination of different functionalities within one 3D scaffold. Here, hFF03 and its variants were systematically studied first as pure compounds.

**Peptide structure and scaffold formation**

**Analysis of peptide structure**

To build up different mixtures of the hydrogels, the peptides were mixed out of stock solutions in hexafluoro isopropanol which allows residue-free evaporation.[28] After evaporation the peptides were dissolved in Dulbecco's phosphate buffered saline (Lonza, w/o $Mg^{2+}$, $Ca^{2+}$). If necessary, pH was adjusted to 7.4. This simple approach allows to tailor different scaffolds by varying the peptide content, the presented ligand type and the presented ligand densities through the whole three-dimensional structure by mixing the unassembled peptides before sample preparation. Fiber formation will initially take place by dissolving the peptides in buffer solution or cell culture medium, respectively. Most of the gels directly built up self-supporting gels within the incubation time of 30 minutes. Hydrogel formation was tested by inverting the sample vials for 30 min at room temperature. This simple test is commonly used to evaluate gel formation of peptides.[21,29] Gel formation was also tested at 37°C and showed no melting effect for all mixtures. In addition, hFF03 and 1 % of hFF03-K17-Man, hFF03 and 5 % of hFF03-K17-RGD and a combination of both (hFF03 + 1 % hFF03-K17-Man + 5 % hFF03-K17-RGD) were studied to test tolerance of cells against ligand type and concentration. 1 % Mannose-bearing hFF03 and 5 % RGD-bearing hFF03 were chosen to nearly adapt their original contents in native ECMs. All pure peptide hydrogels and hydrogel mixtures were tested in 0.15 wt%, 0.30 wt% and 0.50 wt% peptide content.
The secondary structure of the peptide library was studied by CD spectroscopy, the presence of fibers was detected by Cryo-TEM. Network formation and stiffness parameters were recorded by rheological and SANS experiments.

**Circular Dichroism**

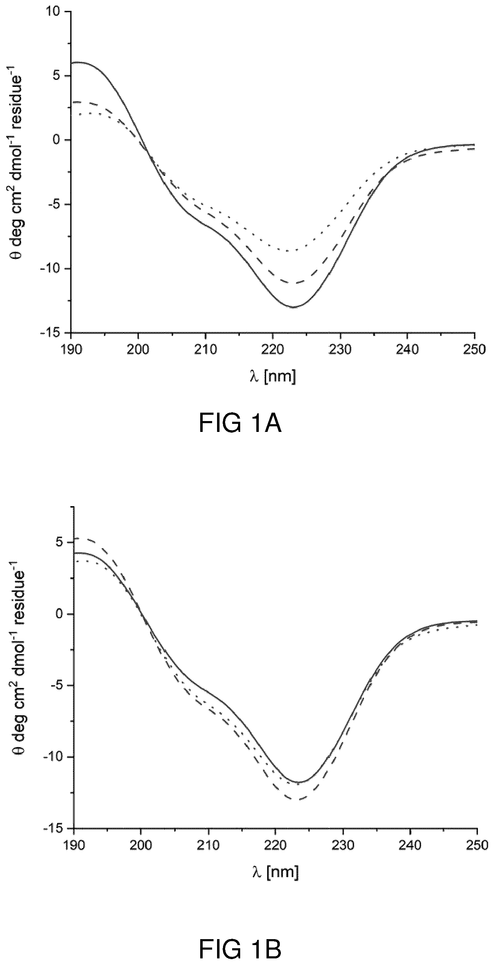

CD spectra were recorded at 20°C and 37°C for all peptides and compositions. hFF03 and its functionalized variants and mixtures thereof show CD-spectra characteristic for an α-helical secondary structure with typical minima around 208 nm and 222 nm as well as a maximum at 195 nm with minimal differences between the studied samples (see Figures 1A and 1B). In Figure 1A, the solid line refers to hFF03, the dashed line refers to hFF03-K17-Man and the dotted line refers to hFF03-K17-RGD. In Figure 1B, the solid line refers to hFF03 + 1 % hFF03-K17-Man + 5 % hFF03-K17-RGD, the dashed line refers to hFF03 + 1 % hFF03-K17-Man, and the dotted line refers to hFF03 + 5 % hFF03-K17-RGD. The CD spectra were normalized according to the path length of the used measuring cuvette, peptide concentration and number of amide bonds.
The minimum around 222 nm is of higher intensity than the minimum around 208 nm for all samples indicating the formation of higher ordered structures .[30] The intensities of CD spectra subsides in the order hFF03, hFF03 + 1 % hFF03-K17-Man, hFF03 + 5 % hFF03-K17-RGD, hFF03 + 1 % hFF03-K17-Man + 5 % hFF03-K17-RGD, hFF03-K17-Man, hFF03-K17-RGD, which is considered to be an indicator for disturbed fiber formation due to the incorporation of peptide and carbohydrate ligands as also previously discussed for modified variants of the peptide scaffold FF03.[11]

**Cryo TEM**

The morphology of the peptide hydrogels was determined by 0.15 wt% hydrogels. Cryo-TEM images showed that the gels are consisting of a homogenous network of peptide fiber bundles with a diameter of 3 nm. Additionally, in the tested hydrogel mixture composed of hFF03 + 1 % hFF03-K17-Man + 5 % hFF03-K17-RGD vesicular inclusions are found within the network structure of the hydrogel. These inclusions might result of hetero assembled peptide fibers as cryo-TEM of the pure functionalized peptide hydrogels hFF03-K17-Man and hFF03-K17-RGD showed no vesicular inclusions over their whole three-dimensional structure.

**Rheological Characterization**

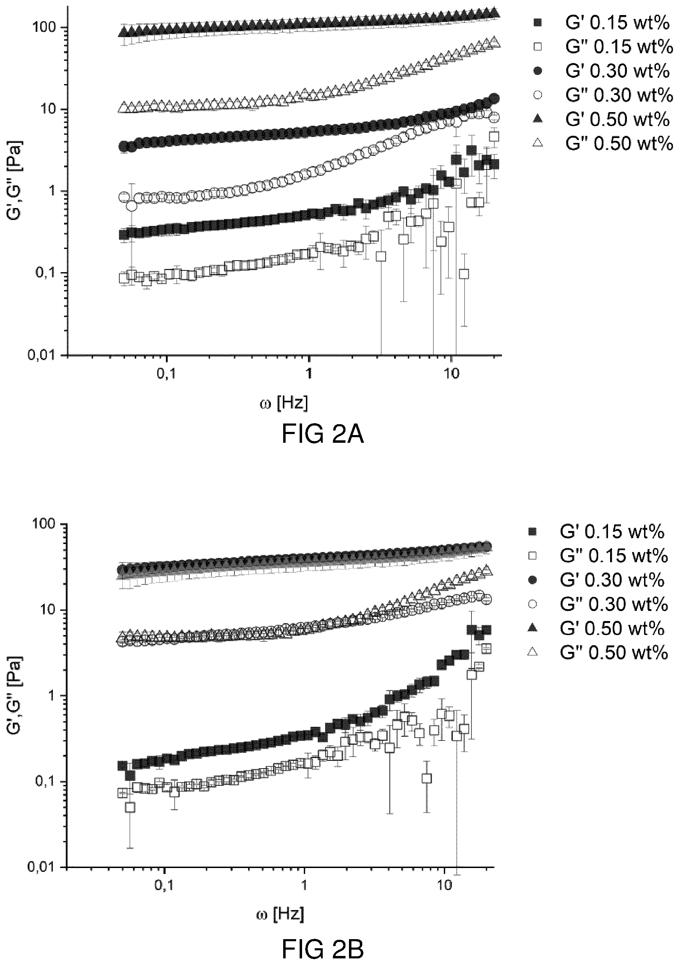

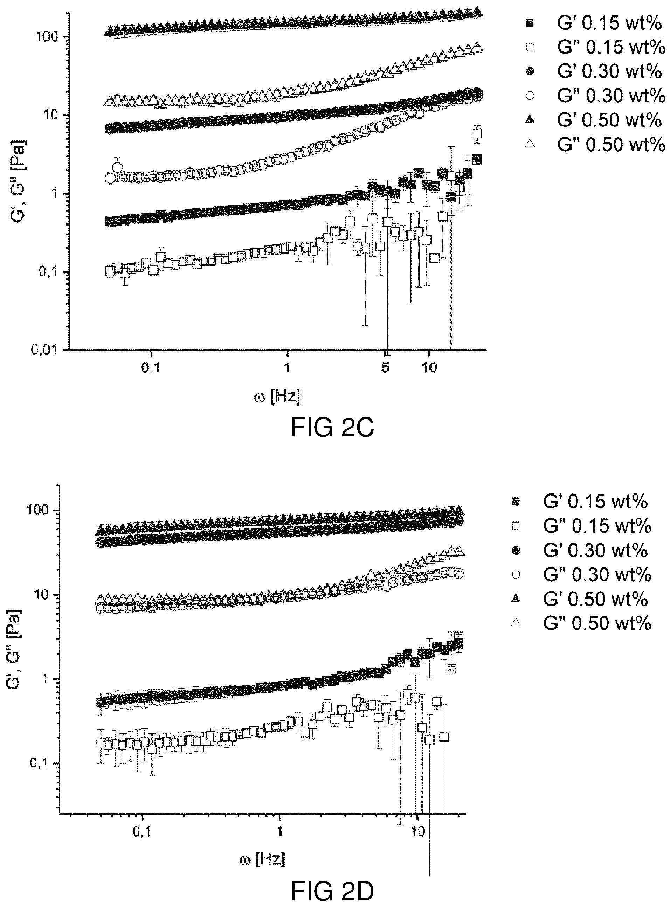

In order to compare the stiffness, respectively the elastic and viscous properties of the hydrogels, which are the critical parameters within the context of biological or medical application, the pure peptide samples (with and without decoration by a peptide or a carbohydrate ligand) as well as mixtures thereof were investigated by oscillatory rheology. To ensure that the frequency dependent measurements are done within the linear viscoelastic (LVE) regime an amplitude sweep in the deformation mode was performed prior to each experiment, yielding the amplitude to be fixed at a deformation of 2 % for all experiments. From the subsequent frequency sweeps in the range of 0.05 to 20 Hz it was possible to determine the loss and storage moduli G' and G" reflecting the elastic and viscous properties, respectively. From all experiments it was found that G' is dominating over G" for the investigated range of frequencies, which means that the hFF03-based system shows pronounced elastic properties. The corresponding data sets for the hFF03 and decorated analogues are presented in Figures 2A and 2C and the data for the mixed peptide systems are presented in in Figures 2B and 2D.

Figure 2A shows the moduli G' and G" of hFF03 for $T_1$ = 25 °C (298 K) for different peptide concentrations. Figure 2B shows the moduli G' and G" of hFF03 +1% hFF03-K17-Man + 5% hFF03-K17-RGD for different peptide concentrations at $T_1$ = 25 °C (298 K). Figure 2C shows the moduli G' and G" of hFF03 for T2 = 37 °C (310 K) for different peptide concentrations. Figure 2D shows the moduli G' and G" of hFF03 +1 % hFF03-K17-Man + 5% hFF03-K17-RGD for different peptide concentrations at $T_1$ = 37 °C (310 K).

To further characterize the frequency-dependent properties of the system the phase angle (loss factor) was taken as a good reference to classify the gels. The phase angle is defined according to the following equation 1:

$$\tan(\delta) = \frac{G'(\omega)}{G''(\omega)} \tag{1}$$

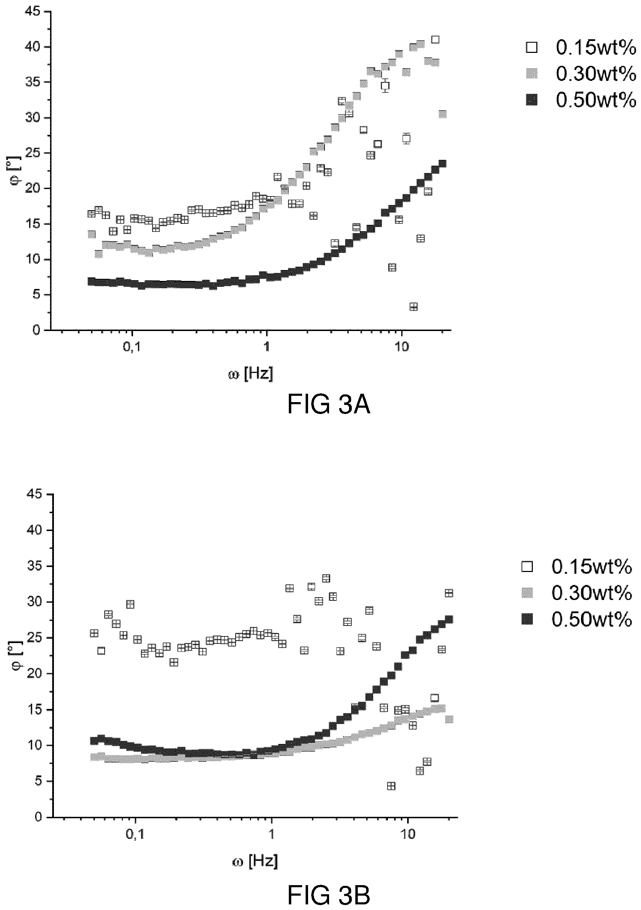

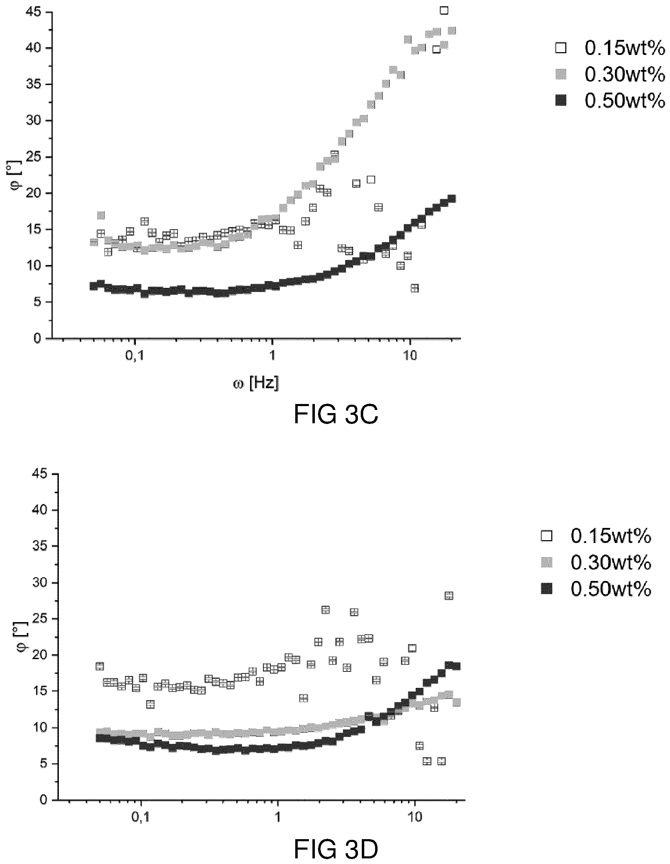

The phase angles of the peptide and peptide mixtures and at different concentrations are exemplary shown in Figures 3A (hFF03) and 3B (hFF03 + 1 % hFF03-K17-Man + 5 % hFF03-K17-RGD) for $T_1$ = 25°C and Figures 3C (hFF03) and 3C (hFF03 + 1 % hFF03-K17-Man + 5 % hFF03-K17-RGD) for T2 = 37°C, which is the relevant temperature in the context of medical and biological applications. It was found that the phase angle is increasing with increasing frequencies, indicating that the underlying system is behaving like a viscoelastic solid or a gel.[31,32] Further, a concentration dependent trend is observed, i.e., the phase angle is decreasing with increasing concentration, which is taken as indication for a stronger gelation at higher concentrations.

Usually, the so-called Kelvin-Voigt model is appropriate to describe the frequency dependent moduli G' and G" within a viscoelastic solid or gel-like material. According to this model, the elastic modulus G' is a constant related to the plateau modulus and G" is linearly depending on the frequency with the viscosity of the material being the corresponding proportionality constant.[32] However, in the system under investigation this approximation is not suitable to describe the data, because neither G' is a constant nor G" is purely linearly depending on the frequency. Due to this fact, the so-called fractional Kelvin-Voigt model was employed. Within this model the moduli are described according to the following equation 2:

$$G'(\omega) = E_1 \omega^\alpha \cos\left(\frac{\pi\,\alpha}{2}\right) + E_2$$
$$G''(\omega) = E_1 \omega^\alpha \sin\left(\frac{\pi\,\alpha}{2}\right) \tag{2}$$

Details for the derivation of these quantities are given elsewhere.[32,33] In equation 2 $E_1$ is a fractional viscosity in Pa s$^\alpha$ depending on the fractional exponent $\alpha$ (0 < $\alpha$ < 1) and E2 is the static load, equivalent to the plateau modulus (in the high frequency limit). Using this description, it is possible to model G' in an appropriate manner for the whole frequency range, while for G" the approximation only in the high frequency regime is possible, which might be attributed to inertia effects at low frequencies. The static load E2 is used to determine the crosslinking or overlapping density $N_c$ as well as an average mesh size $\xi$ through the following equation 3[34]:

$$N_C = \frac{E_2}{kT} = \frac{1}{\xi^3} \qquad (3)$$

Here, k is the Boltzmann constant and T is the absolute temperature. The results for $\xi$ are listed in Table 2 for both examined temperatures.

Table 2. Average mesh size $\xi$ and static load $E_2$ as determined from fitting the data presented in Figures 2B and 2D by the expressions for moduli given in equation 2, which are derived from the fractional Kelvin-Voigt model. The indices refer to the corresponding temperature $T_1 = 25°C$ and $T_2 = 37°C$, respectively.

| Name | c (wt%) | $E_2^{(1)}$ (Pa) | $\xi_1$ (nm) | $E_2^{(2)}$ (Pa) | $\xi_2$ (nm) |
|---|---|---|---|---|---|
| hFF03 | 0.15 | 0.29 | 242.04 | 0.45 | 211.72 |
| | 0.30 | 3.65 | 104.04 | 6.00 | 89.34 |
| | 0.50 | 95.20 | 35.09 | 57.24 | 42.12 |
| hFF03-K17-Man | 0.15 | 2.66 | 115.62 | 6.08 | 88.94 |
| | 0.30 | 0.94 | 163.76 | 4.32 | 99.69 |
| | 0.50 | 6.03 | 88.04 | 15.28 | 65.42 |
| hFF03-K17-RGD | 0.15 | 1.63 | 136.26 | 1.33 | 147.71 |
| | 0.30 | 8.78 | 77.66 | 15.15 | 65.60 |
| | 0.50 | 37.55 | 47.84 | 39.09 | 47.83 |
| hFF03 + 1 % hFF03-K17-Man | 0.15 | 0.27 | 246.86 | 0.33 | 235.55 |
| | 0.30 | 8.23 | 79.34 | 20.17 | 59.64 |
| | 0.50 | 22.44 | 56.80 | 19.42 | 60.39 |
| hFF03 + 5 % hFF03-K17-RGD | 0.15 | 1.67 | 135.16 | 2.39 | 121.41 |
| | 0.30 | 17.48 | 61.73 | 23.26 | 56.87 |
| | 0.50 | 24.21 | 55.38 | 32.11 | 51.07 |
| hFF03 + 1 % hFF03-K17-Man + 5 % hFF03-K17-RGD | 0.15 | 0.17 | 290.75 | 0.60 | 192.25 |
| | 0.30 | 16.97 | 62.34 | 16.61 | 63.63 |
| | 0.50 | 32.27 | 50.32 | 39.68 | 47.60 |

Comparing the values for the mesh size presented in Table 2, it is found that for almost all samples investigated, they are decreasing with increasing concentration, which is reasonable due to the fact that the number density of peptides is increased such that the overlapping areas or mesh sizes, respectively, are getting smaller. Further, it is notable that the mesh sizes at both temperatures, 25°C and 37°C, are of the same order of magnitude and follow the same trends.

**Small Angle Neutron Scattering (SANS)**

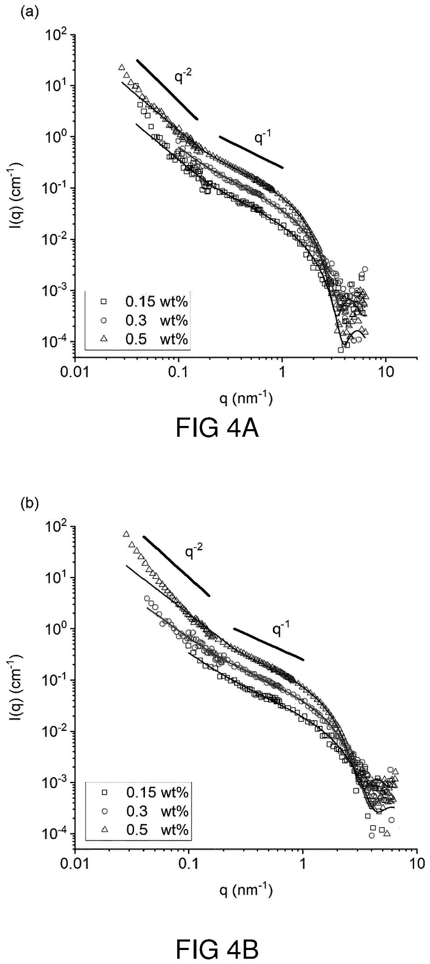

In order to get a more detailed insight into the aggregation behavior of hFF03 and its decorated analogues (hFF03-K17-Man and hFF03-K17-RGD), SANS measurements were performed for different peptide concentrations (0.15, 0.3 and 0.5 wt%). Further, mixtures of the pure and the decorated peptides were investigated. The scattering patterns for hFF03 without decoration and hFF03 + 1 % hFF03-K17-Man + 5 % hFF03-K17-RGD as function of the concentration are shown in Figures 4A (hFF03) and 4B (hFF03 + 1 % hFF03-K17-Man + 5 % hFF03-K17-RGD), respectively. The solid lines represent fits with a form factor model for flexible cylinders as suggested by Pedersen and Schurtenberger.[35]

Comparing the intensities of the scattering data presented in **Fehler! Verweisquelle konnte nicht gefunden werden.** a well-defined concentration trend can be observed, i.e., the intensity is increasing with increasing concentration for the investigated q-range. A direct comparison of the scattering intensities of the peptide hydrogels grouped by the three concentrations yields no difference between the pure and the decorated peptides.

For all scattering patterns a change in slope from $q^{-1}$ at intermediate q values to $q^{-2}$ at low q values is taken as an indication for the formation of flexible, rod-like structures[35]. These structures are usually characterized by the persistence length $L_p$ and the cross-sectional radius $R_{cs}$. In order to determine the latter one, the scattering data in the intermediate q regime are approximated by a modified Guinier approximation,[36,37] given by the following equation 4:

$$I(q) = \frac{A}{q} \cdot \exp\left(-\frac{q^2 R_{g,m}^2}{2}\right) \text{ with } R_{g,m}^2 = \frac{R_{cs}^2}{2}. \tag{4}$$

In equation 4 the pre-factor A on the right-hand side of the equation denotes the forward scattering intensity per unit length. The scattering data in the high q regime is approximated by the generalized Porod law according to the following equation 5:

$$I(q) = \frac{C_1}{q^n} + C_0, \tag{5}$$

In this context, $C_1$ is the Porod scaling constant, $C_0$ accounts for the (incoherent) background and n = 6 - D yields the information of the dimensionality of the scattering objects. If n = 4 the surface of the scattering particle is assumed to be smooth, whereas in case of n being between 3 and 4 the surface of the scattering object is assumed to be rough, i.e. a surface fractal is present. For polymer systems the Porod slope n is related to the excluded volume parameter v, that is n = 1/v: A slope of n = 2 corresponds to a Gaussian chain in a dilute system and n = 3 corresponds to collapsed polymer coils. If the slope is between 2 and 3 the underlying system can be taken as a mass fractal, which is the case, e.g., in networks or branched systems.[38] The results of applying equations 4 and 5 to the scattering data are summarized in Table 3. The Porod exponents of almost all samples hint for a mass fractal structure, except the concentrations 0.3 wt% and 0.5 wt% of the hFF03 decorated with mannose. Here, the Porod slope is greater than three, which is clear indication that in this case the underlying structure has a surface fractal. Comparing the cross-sectional radii obtained from fitting the intermediate q regime with equation 4 all values are around 1 nm, which is in good agreement with the results from TEM.

Table 3. Fit results for the Porod exponent n and the cross-sectional radius $R_{cs}$ obtained from applying equations 4 and 5 to the scattering data. Persistence length and cross-sectional radius obtained by fitting the data with the Pedersen-Schurtenberger model for flexible, rod-like structures.[35]

| Name | c (wt%) | n | $R_{cs}$ (nm) | $L_p$ (nm) | $R_{cs}$ (nm) |
|---|---|---|---|---|---|
| hFF03 | 0.5 | 2.79 | 1.30 | 9.81 | 1.14 |
| | 0.3 | 2.53 | 1.04 | 13.54 | 0.99 |
| | 0.15 | 2.61 | 1.01 | 9.20 | 0.96 |
| hFF03-K17-Man | 0.5 | 3.16 | 1.24 | 7.49 | 1.18 |
| | 0.3 | 3.48 | 1.18 | 8.94 | 1.09 |
| | 0.15 | 2.57 | 1.05 | 8.42 | 1.01 |
| hFF03-K17-RGD | 0.5 | 2.47 | 1.11 | 8.95 | 1.02 |
| | 0.3 | 1.99 | 1.30 | 9.96 | 1.09 |
| | 0.15 | 1.41 | 0.74 | 10.41 | 1.01 |
| hFF03 + 1 % hFF03-K17-Man | 0.5 | 3.35 | 1.16 | 9.10 | 1.14 |
| | 0.3 | 1.97 | 0.78 | 12.68 | 0.79 |
| | 0.15 | 1.71 | 0.94 | 10.44 | 0.86 |
| hFF03 + 5 % hFF03-K17-RGD | 0.5 | 3.46 | 1.16 | 6.96 | 1.14 |
| | 0.3 | 2.38 | 0.99 | 14.15 | 0.96 |
| | 0.15 | 2.51 | 1.03 | 9.80 | 0.98 |
| hFF03 + 1 % hFF03-K17-Man + 5 % hFF03-K17-RGD | 0.5 | 3.26 | 1.16 | 6.59 | 1.14 |
| | 0.3 | 2.28 | 1.03 | 12.27 | 0.97 |
| | 0.15 | 2.51 | 0.91 | 10.70 | 0.84 |

For a more accurate description of the scattering data, the intensities were approximated by a form factor model based on the flexible cylinder model as implemented in SasView.[40] This model originates from simulations done by Pedersen and Schurtenberger on semiflexible polymers, which take into account excluded volume effects.[35] The scattering length density of the peptides is assumed to be $3 \cdot 10^{10}$ cm$^{-2}$.[39] Comparing the cross-sectional radii obtained from the modified Guinier approximation and the Pedersen-Schurtenberger model, they are found to be in good agreement. Further, it is notable that the persistence length of the peptides is for all samples in the same order of magnitude and on average it is around 10 nm. Assuming the peptide chains to be fully stretched, they are found to be on average 17.23 nm, which is in good agreement with the determined persistence length.

## Evaluation of toxicity of different peptide hydrogels

To enhance biocompatibility of the studied peptides, trifluoroacetate counter ions were exchanged for chloride ions by means of treatment with diluted HCl according to established protocols.[41] Cytotoxicity of all peptide candidates was studied to evaluate optimal peptide content and compositions of the proposed extracellular matrix mimics. Cytotoxicity of all hydrogel candidates was studied in a three days cell culture by culturing embryonic mouse fibroblast cell line NIH/3T3 on hydrogel candidates. Different standard compounds served as control. One control experiment was done by growing cells in medium without a hydrogel component. Furthermore, the commercially available and commonly used three-dimensional cell culture scaffold Matrigel®, which represents a mixture of different ECM proteins of the Engelbreth-Holm-Swarm mouse sarcoma, was used as negative control to compare viability of seeded cells on a three-dimensional substrate. Sodium dodecyl sulfate (SDS) was used to establish controlled cell death as positive control.

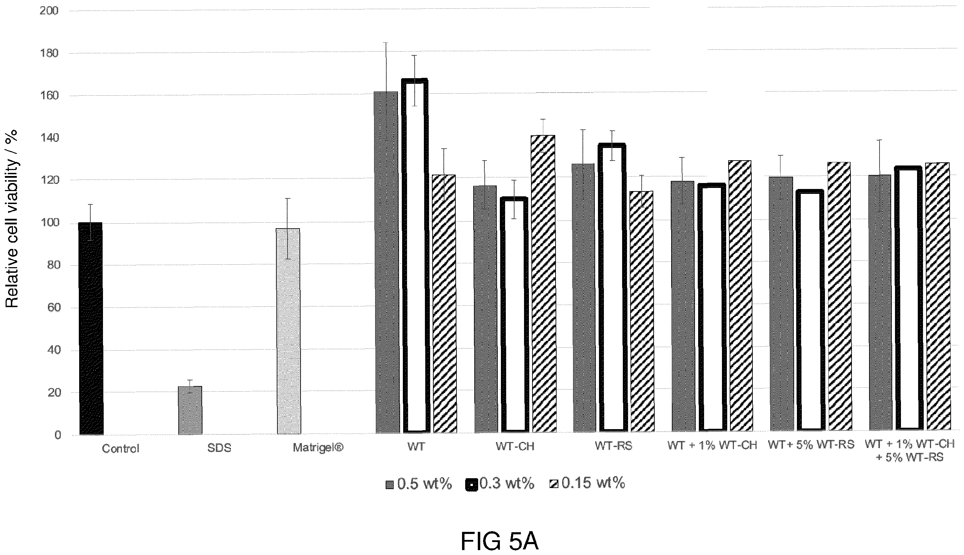

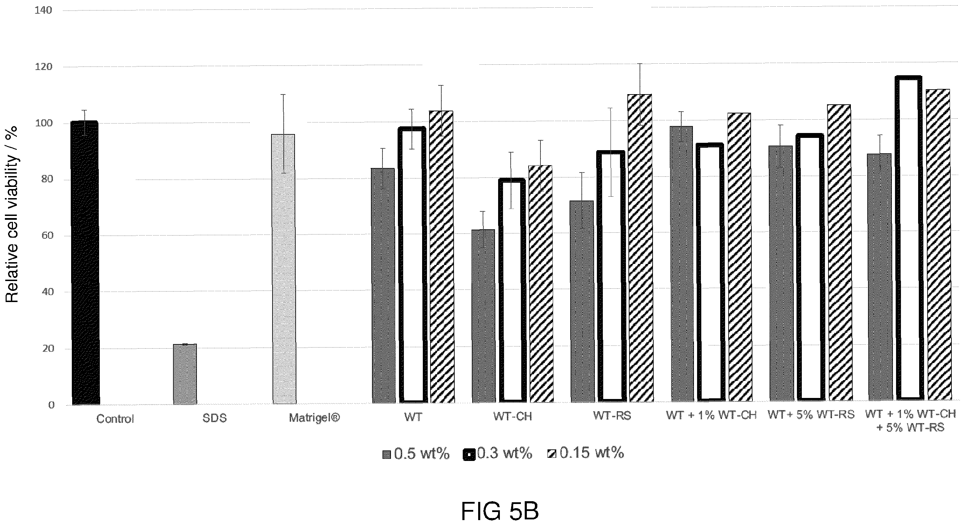

Figures 5A and 5B show the resulting viability profile of seeded NIH3T3-cells on peptide hydrogels after 24 h (Figure 5A) and after 72 h (Figure 5B).
All tested hydrogel candidates initiated an increased viability of seeded cells after 24 h. Compared to both controls (medium and SDS), NIH/3T3 cells cultured on peptide hydrogels show a generally increased viability after 24 h. Especially the undecorated hFF03 hydrogel at 0.3 wt% was tolerated best by the cells resulting in viability values higher than 160 % compared to the control. Cells cultured on each peptide hydrogel mixture showed viabilities in the same range with differences up to 10 %.

## Conclusion

Herein described is the development of a homomeric coiled coil-based peptide hydrogel which enables the presentation of biologically relevant ligands such as small peptide ligands and carbohydrates. The herein described methodology enables the development of tuneable peptide hydrogels with regard to composition and density of presented ligands which are necessary for directing cellular behaviour like proliferation and differentiation. By mixing undecorated and ligand decorated peptides 3D constructs are developed which were tested with regard to their structural and mechanical properties as well as initial cytotoxicity. Structural investigations by cryo-TEM and neutron scattering experiments revealed that the tested systems are composed of fibrillar networks. The mechanical stability of the hydrogels was tested by oscillatory rheology and revealed increasing stiffness by increasing peptide concentration. Initial cytotoxicity tests showed an aproved viability of tested seeded cell population on peptide hydrogel candidates. The results confirm the possibility of tuning three-dimensional cell-culture substrates for a variety of biological applications by mixing different ligand bearing peptides. In contrast to *ex vivo* materials fully synthetic materials such as the herein presented newly designed peptide hydrogels offer the advantage of enhanced reproducibility regarding synthesis and characterization without batch-to-batch variability. Different ligand-bearing peptides provide a large toolbox yielding a great variety of possible combinations to design tailored synthetic ECM mimics for favored applications like tissue engineering.

## List of references cited in the preceding sections or being otherwise considered relevant

[1] K. S. Hellmund, B. Koksch, Front. Chem. 2019, 7, 172.
[2] K. S. Hellmund, Coiled-Coil Based 3D Scaffolds as Highly Specialized Biological Microenvironments, Freie Universität Berlin, 2020.
[3] S. Lou, X. Wang, Z. Yu, L. Shi, Adv. Sci. 2019, 1802043, DOI 10.1002/advs.201802043.
[4] P. Burkhard, J. Stetefeld, S. V. Strelkov, Trends Cell Biol. 2001, 11, 82-88.
[5] E. Wolf, P. S. Kim, B. Berger, Protein Sci. 1997, 6, 1179-1189.
[6] D. N. Woolfson, Adv. Protein Chem. 2005, 70, 79-112.
[7] J. A. Falenski, U. I. M. Gerling, B. Koksch, Bioorg. Med. Chem. 2010, 18, 3703-3706.
[8] K. Pagel, B. Koksch, Curr. Opin. Chem. Biol. 2008, 12, 730-739.

[9] K. Pagel, S. C. Wagner, R. R. Araghi, H. Von Berlepsch, C. Böttcher, B. Koksch, Chem. - A Eur. J. 2008, 14, 11442-11451.

[10] A. N. Lupas, M. Gruber, in Adv. Protein. Chem., 2005, pp. 37-38.

[11] E. Zacco, C. Anish, C. E. Martin, H. v. Berlepsch, E. Brandenburg, P. H. Seeberger, B. Koksch, Biomacromolecules 2015, 16, 2188-2197.

[12] E. Zacco, J. Hütter, J. L. Heier, J. Mortier, P. H. Seeberger, B. Lepenies, B. Koksch, ACS Chem. Biol. 2015, 10, 2065-2072.

[13] R. S. Hodges, Biochem. Cell Biol. 1996, 74, 133-154.

[14] J. R. Litowski, R. S. Hodges, **2002**, 277, 37272-37279.

[15] P. B. Harbury, T. Zhang, P. S. Kim, T. Alber, Science. 1993, 262, 1401-1407.

[16] J. M. Fletcher, A. L. Boyle, M. Bruning, Sg. J. Bartlett, T. L. Vincent, N. R. Zaccai, C. T. Armstrong, E. H. C. Bromley, P. J. Booth, R. L. Brady, et al., ACS Synth. Biol. 2012, 1, 240-250.

[17] E. F. Banwell, E. S. Abelardo, D. J. Adams, M. a Birchall, A. Corrigan, A. M. Donald, M. Kirkland, L. C. Serpell, M. F. Butler, D. N. Woolfson, Nat. Mater. 2009, 8, 596-600.

[18] N. Mehrban, E. Abelardo, A. Wasmuth, K. L. Hudson, L. M. Mullen, A. R. Thomson, M. A. Birchall, D. N. Woolfson, Adv. Healthc. Mater. 2014, 3, 1387-1391.

[19] N. Mehrban, B. Zhu, F. Tamagnini, F. I. Young, A. Wasmuth, K. L. Hudson, A. R. Thomson, M. A. Birchall, A. D. Randall, B. Song, et al., ACS Biomater. Sci. Eng. 2015, 1, 431-439.

[20] S. A. Potekhin, T. N. Melnik, V. Popov, N. F. Lanina, A. A. Vazina, P. Rigler, A. S. Verdini, G. Corradin, A. V. Kajava, Chem. Biol. 2001, 8, 1025-1032.

[21] E. F. Banwell, E. S. Abelardo, D. J. Adams, M. a Birchall, A. Corrigan, A. M. Donald, M. Kirkland, L. C. Serpell, M. F. Butler, D. N. Woolfson, Nat. Mater. 2009, 8, 596-600.

[22] M. D. Pierschbacher, E. Ruoslahti, Proc. Natl. Acad. Sci. U. S. A. 1984, 81, 5985-5988.

[23] K. Luder, K. Kulkarni, H. W. Lee, R. E. Widdop, M. P. Del Borgo, M. I. Aguilar, Chem. Commun. 2016, 52, 4549-4552.

[24] J. R. Klim, L. Li, P. J. Wrighton, M. S. Piekarczyk, L. L. Kiessling, **2010,** 7, DOI 10.1038/nmeth.1532.

[25] M. MILLIPORE, Novabiochem (R) Guide to Selection of Building Blocks, n.d**.**

[26] E. Ruoslahti, Annu. Rev. Cell Dev. Biol. 1996, 12, 697-715.

[27] C. Cha, W. B. Liechty, A. Khademhosseini, N. A. Peppas, ACS Nano 2012, 6, 9353-9358.

[28] E. Brandenburg, H. V. Berlepsch, B. Koksch, Mol. Biosyst. 2012, 8, 557-564.

[29] M. Lian, X. Chen, Y. Lu, W. Yang, **2016,** 6-12.

[30] M. J. Pandya, G. M. Spooner, M. Sunde, J. R. Thorpe, A. Rodger, D. N. Woolfson, Biochemistry 2000, 39, 8728-8734.

[31] R. L. Bagley, P. J. Torvik, AIAA J. 1983, 21, 741-748.

[32] L. B. Eldred, W. P. Baker, A. N. Palazotto, AIAA J. 1995, 33, 547-550.

[33] R. Lewandowski, B. Chorazyczewski, Comput. Struct. 2010, 88, 1-17.

[34] M. Doi, S. F. Edwards, The Theory of Polymer Dynamics, Oxford University Press, 1986**.**

[35] J. S. Pedersen, P. Schurtenberger, Macromolecules 1996, 29, 7602-7612.

[36] R. P. Hjelm, P. Thiyagarajan, H. Alkan-Onyuksel, J. Phys. Chem. 1992, 96, 8653-8661.

[37] R. P. Hjelm, C. Schteingart, A. F. Hoffmann, D. S. Sivia, J. Phys. Chem. 1995, 99, 16395-16406.

[38] B. Hammouda, J. Appl. Crystallogr. 2010, 43, 716-719.

[39] B. Jacrot, Reports Prog. Phys. 1976, 39, 911-953.

[40] "https://www.sasview.org/publications/," **n.d.**

[41] V. V Andrushchenko, H. J. Vogel, E. J. Prenner, J. Pept. Sci. 2007, 13, 37-43.

[42] U. Keiderling, Appl. Phys. A Mater. Sci. Process. 2002, 74, 1455-1457.

[43] A. Brûlet, D. Lairez, A. Lapp, J.-P. Cotton, J. Appl. Crystallogr. 2007, 40, 165-177.

[44] US 2011/0189284 A1

[45] Costas A. Lyssiotis,* Luke L. Lairson, Anthony E. Boitano, Heiko Wurdak, Shoutian Zhu, Peter G. Schultz, Angew. Chem. 2011, 123, 210 - 256.

**Claims**

1. Peptide, comprising n heptad repeat units having a primary structure of LXXLKKE (SEQ ID NO. 1), wherein in one heptad repeat unit one of the X residues is A and the other of the X residues is A, F, Y or W, wherein in n-1 heptad repeat units both X residues are A, wherein n is 2 to 10.

2. Peptide according to claim 1, **characterized in that** the heptad repeat units have a primary structure of LAXLKKE (SEQ ID NO. 2), wherein in one heptad repeat unit the X residue is A, F, Y or W, wherein in n-1 heptad repeat units the X residue is A.

3. Peptide according to claim 1, **characterized in that** the heptad repeat units have a primary structure of LXALKKE (SEQ ID NO. 3), wherein in one heptad repeat unit the X residue is A, F, Y or W, wherein in n-1 heptad repeat units the X residue is A.

4. Peptide according to claim 1, **characterized in that** the heptad repeat units have a primary structure of LAALKKE (SEQ ID NO. 4).

5. Peptide according to any of the preceding claims, **characterized in that** the peptide comprises 1 to 10 additional amino acids.

6. Peptide according to claim 5, **characterized in that** the additional amino acids comprise additional N-terminal amino acids and additional C-terminal amino acids, wherein a number of the additional N-terminal amino acids is greater than a number of the additional C-terminal amino acids.

7. Peptide according to any of the preceding claims, **characterized in that** all heptad repeat units are directly linked to each other.

8. Peptide according to any of the preceding claims, **characterized in that** the peptide has a primary structure of LKKELXXLKKELXXLKKELXXLKKEL (SEQ ID NO. 5), wherein one of the X residues is A, F, Y or W, wherein all other of the X residues are A.

9. Peptide according to any of the preceding claims, **characterized in that** the peptide has a primary structure of LKKELAALKKELAALKKELAALKKEL (SEQ ID NO. 6).

10. Peptide according to any of the preceding claims, **characterized in that** the peptide comprises at least one ligand molecule covalently bound to any of the amino acids of the peptide, wherein the ligand molecule is chosen from the group consisting of peptides, molecules comprising an epitope, hydrocarbons, carbohydrates, and pharmaceutically active compounds.

11. Hydrogel, comprising a plurality of molecules of a peptide according to any of the preceding claims.

12. Hydrogel according to claim 11, **characterized in that** all peptide molecules in the hydrogel have the same primary structure.

13. Peptide according to any of claims 1 to 10 or hydrogel according to claim 11 or 12 for use in therapy or surgery.

14. Use of a peptide according to any of claims 1 to 10 or of a hydrogel according to claim 11 or 12 for manufacturing cosmetics or as extracellular matrix for in vitro applications.

15. Method for manufacturing a peptide according to any of claims 1 to 10 comprising the following steps:

a) synthesizing a peptide by solid phase peptide synthesis on a solid phase peptide synthesis resin, the peptide comprising n heptad repeat units having a primary structure of LXXLKKE (SEQ ID NO. 1), wherein in one heptad repeat unit one of the X residues is A and the other of the X residues is A, F, Y or W, wherein in n-1 heptad repeat units both X residues are A, wherein n is 2 to 10;
b) cleaving the synthesized peptide from the solid phase peptide synthesis resin;
c) optionally purifying the synthesized and cleaved peptide.

FIG 1A

FIG 1B

FIG 2A

FIG 2B

FIG 2C

FIG 2D

FIG 3A

FIG 3B

FIG 3C

FIG 3D

(a)

FIG 4A

(b)

FIG 4B

FIG 5A

EP 3 909 969 A1

FIG 5B

Europäisches
Patentamt

European
Patent Office

Office européen
des brevets

# EUROPEAN SEARCH REPORT

Application Number

EP 20 17 4972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | S E FISCHER ET AL: "Controlling cell adhesion to surfaces via associating bioactive triblock proteins", BIOMATERIALS, vol. 28, no. 22, 10 May 2007 (2007-05-10), pages 3325-3337, XP022067460, DOI: 10.1016/J.BIOMATERIALS.2007.03.026 | 1-4,11, 12,14,15 | INV. C07K7/08 C07K14/00 |
| Y | * the whole document, in particular | 10 | |
| A | abstract; figures 1,2; compound CRC * | 5-9,13 | |
| Y | E ZACCO ET AL: "A Self-Assembling Peptide Scaffold for the Multivalent Presentation of Antigens", BIOMACROMOLECULES, vol. 16, no. 7, 4 June 2015 (2015-06-04), pages 2188-2197, XP055738991, DOI: 10.1021/acs.biomac.5b00572 * Table 1; figure 2; compound FF03 * | 10 | |
| T | K S HELLMUND ET AL: "Functionalized peptide hydrogels as tunable extracellular matrix mimics for biological applications", HAL, 8 July 2020 (2020-07-08), page 02893349, XP055738944, Retrieved from the Internet: URL:https://hal.archives-ouvertes.fr/hal-0 2893349/document [retrieved on 2020-10-12] * the whole document * | 1-15 | TECHNICAL FIELDS SEARCHED (IPC) C07K |

The present search report has been drawn up for all claims

| Place of search | Date of completion of the search | Examiner |
|---|---|---|
| Munich | 13 October 2020 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110189284 A1

**Non-patent literature cited in the description**

- **K. S. HELLMUND ; B. KOKSCH.** *Front. Chem.,* 2019, vol. 7, 172
- **K. S. HELLMUND.** Coiled-Coil Based 3D Scaffolds as Highly Specialized Biological Microenvironments. Freie Universität Berlin, 2020
- **S. LOU ; X. WANG ; Z. YU ; L. SHI.** *Adv. Sci.,* 2019, 1802043
- **P. BURKHARD ; J. STETEFELD ; S. V. STRELK-OV.** *Trends Cell Biol.,* 2001, vol. 11, 82-88
- **E. WOLF ; P. S. KIM ; B. BERGER.** *Protein Sci.,* 1997, vol. 6, 1179-1189
- **D. N. WOOLFSON.** *Adv. Protein Chem.,* 2005, vol. 70, 79-112
- **J. A. FALENSKI ; U. I. M. GERLING ; B. KOKSCH.** *Bioorg. Med. Chem.,* 2010, vol. 18, 3703-3706
- **K. PAGEL ; B. KOKSCH.** *Curr. Opin. Chem. Biol.,* 2008, vol. 12, 730-739
- **K. PAGEL ; S. C. WAGNER ; R. R. ARAGHI ; H. VON BERLEPSCH ; C. BÖTTCHER ; B. KOKSCH.** *Chem. - A Eur. J.,* 2008, vol. 14, 11442-11451
- **A. N. LUPAS ; M. GRUBER.** *Adv. Protein. Chem.,* 2005, 37-38
- **E. ZACCO ; C. ANISH ; C. E. MARTIN ; H. V. BERLEPSCH ; E. BRANDENBURG ; P. H. SEEBERGER ; B. KOKSCH.** *Biomacromolecules,* 2015, vol. 16, 2188-2197
- **E. ZACCO ; J. HÜTTER ; J. L. HEIER ; J. MORTIER ; P. H. SEEBERGER ; B. LEPENIES ; B. KOKSCH.** *ACS Chem. Biol.,* 2015, vol. 10, 2065-2072
- **R. S. HODGES.** *Biochem. Cell Biol.,* 1996, vol. 74, 133-154
- **P. B. HARBURY ; T. ZHANG ; P. S. KIM ; T. ALBER.** *Science,* 1993, vol. 262, 1401-1407
- **J. M. FLETCHER ; A. L. BOYLE ; M. BRUNING ; SG. J. BARTLETT ; T. L. VINCENT ; N. R. ZACCAI ; C. T. ARMSTRONG ; E. H. C. BROMLEY ; P. J. BOOTH ; R. L. BRADY et al.** *ACS Synth. Biol.,* 2012, vol. 1, 240-250
- **E. F. BANWELL ; E. S. ABELARDO ; D. J. ADAMS ; M. A BIRCHALL ; A. CORRIGAN ; A. M. DONALD ; M. KIRKLAND ; L. C. SERPELL ; M. F. BUTLER ; D. N. WOOLFSON.** *Nat. Mater.,* 2009, vol. 8, 596-600

- **N. MEHRBAN ; E. ABELARDO ; A. WASMUTH ; K. L. HUDSON ; L. M. MULLEN ; A. R. THOMSON ; M. A. BIRCHALL ; D. N. WOOLFSON.** *Adv. Healthc. Mater.,* 2014, vol. 3, 1387-1391
- **N. MEHRBAN ; B. ZHU ; F. TAMAGNINI ; F. I. YOUNG ; A. WASMUTH ; K. L. HUDSON ; A. R. THOMSON ; M. A. BIRCHALL ; A. D. RANDALL ; B. SONG et al.** *ACS Biomater. Sci. Eng.,* 2015, vol. 1, 431-439
- **S. A. POTEKHIN ; T. N. MELNIK ; V. POPOV ; N. F. LANINA ; A. A. VAZINA ; P. RIGLER ; A. S. VERDINI ; G. CORRADIN ; A. V. KAJAVA.** *Chem. Biol.,* 2001, vol. 8, 1025-1032
- **M. D. PIERSCHBACHER ; E. RUOSLAHTI.** *Proc. Natl. Acad. Sci. U. S. A.,* 1984, vol. 81, 5985-5988
- **K. LUDER ; K. KULKARNI ; H. W. LEE ; R. E. WIDDOP ; M. P. DEL BORGO ; M. I. AGUILAR.** *Chem. Commun.,* 2016, vol. 52, 4549-4552
- **M. MILLIPORE.** *Novabiochem (R) Guide to Selection of Building Blocks*
- **E. RUOSLAHTI.** *Annu. Rev. Cell Dev. Biol.,* 1996, vol. 12, 697-715
- **C. CHA ; W. B. LIECHTY ; A. KHADEMHOSSEINI ; N. A. PEPPAS.** *ACS Nano,* 2012, vol. 6, 9353-9358
- **E. BRANDENBURG ; H. V. BERLEPSCH ; B. KOK-SCH.** *Mol. Biosyst.,* 2012, vol. 8, 557-564
- **M. J. PANDYA ; G. M. SPOONER ; M. SUNDE ; J. R. THORPE ; A. RODGER ; D. N. WOOLFSON.** *Biochemistry,* 2000, vol. 39, 8728-8734
- **R. L. BAGLEY ; P. J. TORVIK.** *AIAA J.,* 1983, vol. 21, 741-748
- **L. B. ELDRED ; W. P. BAKER ; A. N. PALAZOTTO.** *AIAA J.,* 1995, vol. 33, 547-550
- **R. LEWANDOWSKI ; B. CHORAZYCZEWSKI.** *Comput. Struct.,* 2010, vol. 88, 1-17
- **M. DOI ; S. F. EDWARDS.** The Theory of Polymer Dynamics. Oxford University Press, 1986
- **J. S. PEDERSEN ; P. SCHURTENBERGER.** *Macromolecules,* 1996, vol. 29, 7602-7612
- **R. P. HJELM ; P. THIYAGARAJAN ; H. AL-KAN-ONYUKSEL.** *J. Phys. Chem.,* 1992, vol. 96, 8653-8661

- **R. P. HJELM ; C. SCHTEINGART ; A. F. HOFFMANN ; D. S. SIVIA.** *J. Phys. Chem.,* 1995, vol. 99, 16395-16406
- **B. HAMMOUDA.** *J. Appl. Crystallogr.,* 2010, vol. 43, 716-719
- **B. JACROT.** *Reports Prog. Phys.,* 1976, vol. 39, 911-953
- **V. V ANDRUSHCHENKO ; H. J. VOGEL ; E. J. PRENNER.** *J. Pept. Sci.,* 2007, vol. 13, 37-43
- **U. KEIDERLING.** *Appl. Phys. A Mater. Sci. Process.,* 2002, vol. 74, 1455-1457
- **A. BRÛLET ; D. LAIREZ ; A. LAPP ; J.-P. COTTON.** *J. Appl. Crystallogr.,* 2007, vol. 40, 165-177
- **COSTAS A. LYSSIOTIS ; LUKE L. LAIRSON ; ANTHONY E. BOITANO ; HEIKO WURDAK ; SHOU-TIAN ZHU ; PETER G. SCHULTZ.** *Angew. Chem.,* 2011, vol. 123, 210-256

SEQUENCE LISTING

<110> Freie Universität Berlin

<120> Peptide for hydrogel formation

<130> FU208EP

<160> 6

<170> PatentIn version 3.5

<210> 1
<211> 7
<212> PRT
<213> Artificial Sequence

<220>
<223> Synthetic Construct

<220>
<221> VARIANT
<222> (2)..(3)
<223> Xaa can be Ala, Phe, Tyr, or Trp

<400> 1

Leu Xaa Xaa Leu Lys Lys Glu
1               5

<210> 2
<211> 7
<212> PRT
<213> Artificial Sequence

<220>
<223> Synthetic Construct

<220>
<221> VARIANT
<222> (3)..(3)
<223> Xaa can be Ala, Phe, Tyr, or Trp

<400> 2

Leu Ala Xaa Leu Lys Lys Glu
1               5

<210> 3
<211> 7
<212> PRT
<213> Artificial Sequence

<220>
<223> Synthetic Construct

<220>
<221> VARIANT

```
<222>  (2)..(2)
<223>  Xaa can be Ala, Phe, Tyr, or Trp


<400>  3

Leu Xaa Ala Leu Lys Lys Glu
1               5



<210>  4
<211>  7
<212>  PRT
<213>  Artificial Sequence

<220>
<223>  Synthetic Construct


<400>  4

Leu Ala Ala Leu Lys Lys Glu
1               5



<210>  5
<211>  26
<212>  PRT
<213>  Artificial Sequence

<220>
<223>  Synthetic Construct


<220>
<221>  VARIANT
<222>  (6)..(21)
<223>  Xaa can be Ala, Phe, Tyr, or Trp

<400>  5

Leu Lys Lys Glu Leu Xaa Xaa Leu Lys Lys Glu Leu Xaa Xaa Leu Lys
1               5               10              15


Lys Glu Leu Xaa Xaa Leu Lys Lys Glu Leu
            20              25



<210>  6
<211>  26
<212>  PRT
<213>  Artificial Sequence

<220>
<223>  Synthetic Construct

<400>  6

Leu Lys Lys Glu Leu Ala Ala Leu Lys Lys Glu Leu Ala Ala Leu Lys
1               5               10              15


Lys Glu Leu Ala Ala Leu Lys Lys Glu Leu
            20              25
```

15